Europäisches Patentamt

European Patent Office

Office européen des brevets

(11) Publication number: **0 416 846 A2**

# EUROPEAN PATENT APPLICATION

(21) Application number: **90309616.2**

(22) Date of filing: **03.09.90**

(51) Int. Cl.⁵: **A61F 2/12, A61F 2/02**

(30) Priority: **05.09.89 US 402746**

(43) Date of publication of application:
**13.03.91 Bulletin 91/11**

(84) Designated Contracting States:
**CH DE ES FR GB IT LI NL SE**

(71) Applicant: **DOW CORNING WRIGHT CORPORATION**
**P.O. Box 100 5677 Airline Road**
**Arlington Tennessee(US)**

(72) Inventor: **Curtis, James Michael**
**6194 Ivanhoe Road**
**Bartlett, Tennessee(US)**

(74) Representative: **Lewin, John Harvey et al**
**ELKINGTON AND FIFE Beacon House 113 Kingsway**
**London WC2B 6PP(GB)**

(54) Method of manufacturing an implantable article provided with a micropillared surface.

(57) A method is provided for manufacturing an implant such as an envelope for a mammary prosthesis intended for implantation in a human breast which envelope is provided with a surface having outwardly projecting micropillars. The method involves providing a three dimensional mandrel, the outer surface of which has an array of depressions constituting said pillars in negative relief, applying a crosslinkable elastomer-forming liquid dispersion of a polymer such as a polyurethane or polydimethylsiloxane to said mandrel surface, subjecting said mandrel and polymer to an elevated pressure, crosslinking said polymer while under elevated pressure and stripping the resultant elastomeric envelope or other object from said mandrel.

EP 0 416 846 A2

# METHOD OF MANUFACTURING AN IMPLANTABLE ARTICLE PROVIDED WITH A MICROPILLARED SUR-FACE

The invention relates to a method of manufacturing an article such as a mammary prosthesis or other implantable medical device which is provided with a surface having micropillars integral therewith which micropillars project outwardly from the surface to provide an implant having desirable surface characteristics. The process contemplates the use of a mold surface having the desired micropillared structure in negative relief and an elastomer-forming liquid dispersion of a polymer is applied to the surface. The polymer-coated mold is then subjected to an elevated pressure and the polymer is cured, by chain-extension and/or cross-linking to form an elastomer while under an elevated pressure.

It has previously been suggested that the texturing of the outer surface of a mammary or other soft tissue implant may be instrumental in construction of implants that alter the architecture of the tissue interface with the implant and, hence, may have improved resistance to the formation of capsular contracture responses in the tissue surrounding the implant. See, for example, Ion-Beam Microtexturing of Biomaterials; Picha & Siedlak, Medical Device and Diagnostic Industry (April 1984), Canon Publ. Inc. 2416 Wilshire Blvd. Santa Monica, CA. Further examples are described in a master's thesis by Elizabeth Ann Powell of the Department of Biomedical Engineering, Case Western Reserve University entitled "Changes in the Subcutaneous Tissue Response Caused by Implant Compliance and Surface Morphology" (May 1982). Thee is a disclosure of formation of prostheses in which the surfaces contain pillars which were 75 microns in width and 150 microns in height. The micropillars were separated from each other by a distance of 75 microns.

In said thesis a process of forming the micropillared surface is disclosed which comprises pouring a silicone dispersion over a negative textured epoxy mold and then placing the mold in a vacuum in order to draw out air bubbles thus facilitating penetration of the dispersion into the pits of the texture. This process has the disadvantage that it is very difficult to strip the resultant article from the mold without damaging the micropillars. The materials described were two dimensional sheets rather than three dimensional objects of a type necessary for implantation as a viable prosthesis. Commercial production of such devices therefore have not heretofore thought to be practical and a need has remained for improved processes for fabrication of such devices.

Another example in which somewhat larger rigid protubrances were proposed to be incorporated in the outside of a mammary prosthesis to space the tissues away from the surface of the prosthesis is disclosed in U.S. Patent No. 4,531,244 (Hamas) issued July 30, 1985. Said patent does not, however, disclose a commercially feasible method for producing such implants. Further, the protuberances are disclosed as constituting rigid spacers designed to act as spacers for the body tissues surrounding the implant rather than elastomeric micropillars which interact with and alter the response of the surrounding tissues.

A principle object of the present invention is to provide an improved and commercially feasible method for producing micropillared surfaces on three dimensional implantable devices such as transcutaneous catheters, artificial hearts, left ventricle assist devices and other short and long term medical implants such as mammary prostheses and tissue expanders. In general the microtextured surfaces produced in accordance with the present invention may be used in connection with any device in which it is desired to modify the response of body tissues to the implant. It is believed that implants produced in accordance with the present invention have surfaces that alter the architecture of the implant/tissue interface and may have improved resistance to the formation of capsular contracture responses in the scar tissue surrounding the implant.

The objectives of the present invention are achieved by providing an improved process for formation of medical implants from a dispersion of a polymer which is curable, cross-linkable or otherwise reactable and carried in an appropriate liquid medium. In accordance with the present invention, a three-dimensional mandrel is provided which has a surface containing an array of blind microholes therein which have the general shape and dimensions of the desired micropillars in negative relief. Such mandrels are generally formed by such techniques as ion-beam bombardment through an appropriate screen in order to cause a formation of appropriately sized and shaped microholes in the surface of the mandrel, machine drilling or by laser drilling of the holes. The micropillars of the present invention generally have a width of 5-3000 microns but more preferably, for many applications, such as mammary implants, 25-800 microns and a height of approximately 75-3500 microns. The precise dimensions of the micropillars does not form part of the present invention. The preferred micropillar dimensions for any specific application are determined by testing the response of living tissues to particular morphologies. The term "microholes" as used herein refers to blind holes having a diameter of about 5 to 3000 microns and a depth of about 5 to 3500 micros.

Briefly summarized, the method of this invention provides an article for implantation in living tissue which article is provided with a surface having micropillars integral therewith and projecting outwardly from its surface and includes the steps of providing a mold surface having an array of blind microholes thereon, applying an elastomer-forming liquid dispersion of a polymer to the mold surface, subjecting the mold and polymer to an elevated pressure, curing the polymer while under such elevated pressure and stripping the resultant elastomeric article from said mold. The article may then be turned inside out, if, as is the case with most implants, it is desired that the micropillared surface constitutes the exterior of the implant.

The objects, advantages and novel features of the invention will be more fully apparent from the following detailed description when read in conjunction with accompanying drawings wherein:

Figure 1 is a schematic view showing one embodiment of a dispersion applying step in accordance with the present invention,

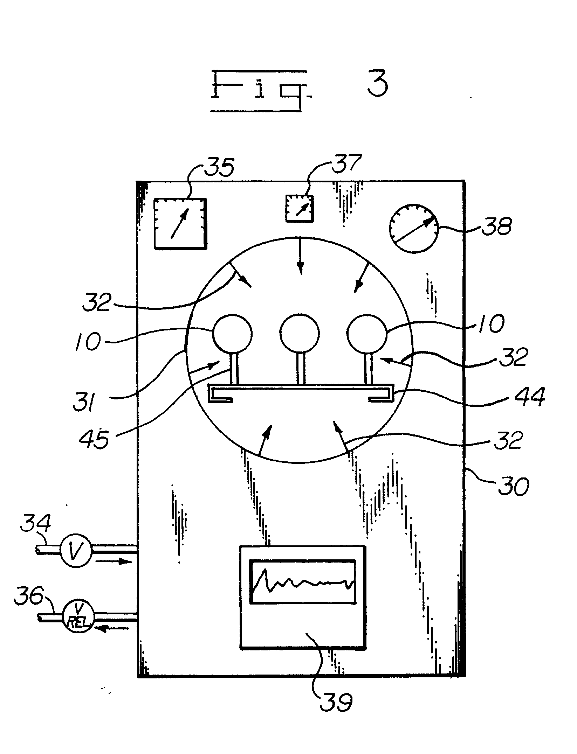

Figure 2 is a schematic view showing a curing step in accordance with the present invention, and

Figure 3 is a greatly enlarged sectional view with parts broken away illustrating a mold stripping step in accordance with the present invention.

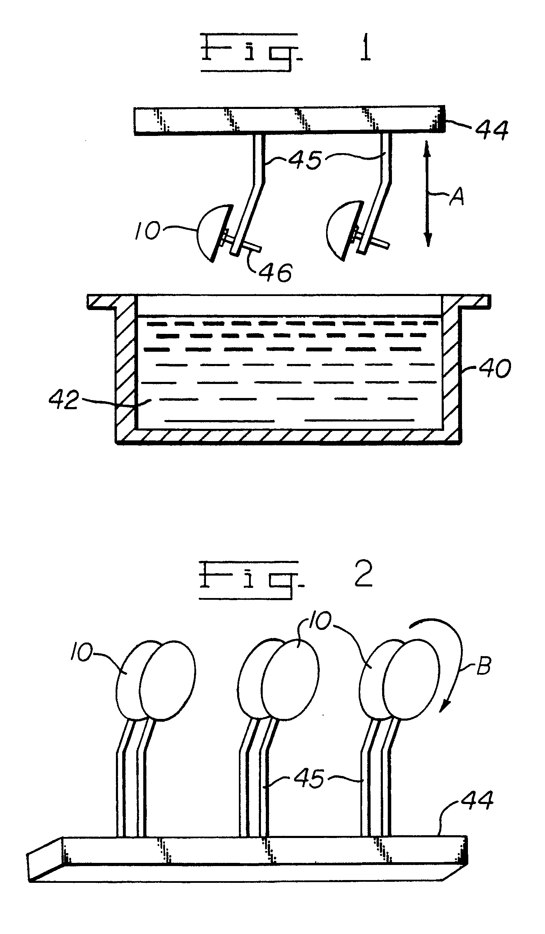

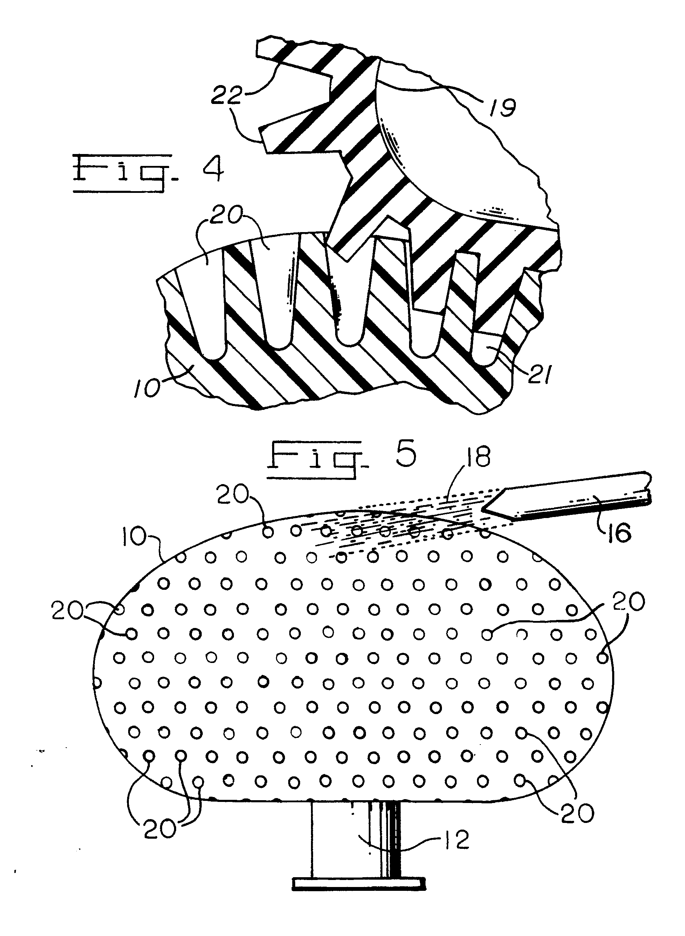

Referring more particularly to Figure 1 there are seen mandrels 10 which are generally formed from a hard resinous polymeric material such as epoxy or polyester (eg. polyethylene terephthalate), polyvinylidene flouride, polyacetal (homo or copolymer), polytetrafluoroethylene, perfluoroethylene or other fluoropolymers. The mandrels have an exterior surface provided with blind microholes 20, best seen in greatly magnified form in Figure 4. Mandrels 10 are preferably mounted on a dripping fixture 44 using arms 45 in conjunction with mounting shafts 46 that are affixed to the rear central portion of each mandrel, a position that will become the posterior portion of prosthesis shells that are formed by the procedure. A polymeric dispersion 42 to the exterior of mandrels 10 is by means of dipping into a tank 40 containing liquid dispersion of the elastomer, as indicated by arrow A. Typically the mandrels are dipped as many as nine times to build up a desired thickness of polymer on the surface thereof and are rotated between dips as indicated by arrow "B" to avoid too much build up on one side of the mandrels due to gravity flow.

Another method of coating the mandrels is by spraying a stream 18 of dispersion onto the mandrels from spray nozzle 16. Such nozzles are typically robotically driven so as to deposit an even layer of polymer on the exterior of a mandrel which is rotated during the spraying process. Any other conventional method of applying a dispersion to the mandrels may be employed, for example, application by rollers, brushes, etc.

Referring to Figure 3 there is seen a pressure oven 30 containing a pressure chamber 31 in which a number of coated mandrels 10 on a supporting tray 44 are placed. Arrows 32 intended to figuratively indicate the elevated pressure within chamber 31. Appropriate inlet and outlet conduits 34 and 36 may be employed to introduce and remove heated pressurized gases from the interior of chamber 31. In accordance with conventional practice temperature controllers 35 and 37 and pressure regulator 38 are preferably provided on the oven. The exact curing temperature is selected in accordance with the conditions needed to cross-link or cure the particular polymer which is coated on mandrels 10. The pressure selected depends on the morphology of the pillars desired and the mandrel microholes. With commercially used dispersions, gauge pressures of 0.3 to 70 kilograms per square centimeter have been found to be operable.

As seen in Figure 4, an implant shell 19 is formed by virtue of drying and cross-linking of the polymeric dispersion 42 or 18. The blind microholes on the surface of mandrel 10 are indicated by numerical 20. It has been found that tiny air pockets 21 are entrapped in the blind microholes 20. The use of elevated pressure during the curing step retains these pockets in a compressed state at the bottom of the blind microholes 20 on the mandrels. It has further been found that relieving of the pressure after curing of the polymer results in the air in pockets 21 tending to push the micropillars 22 out of the microholes 20, thus greatly increasing the ese of stripping of implant shell 19 from mandrel 10. Additionally, the presence of the entrapped air 21 tends to flatten out the tips of pillars 22 which would otherwise tend to be rounded or pointed depending on the contour of the bottom of the microholes. Typically if the blind microholes are formed by electron bombardment or laser drilling, the pit bottoms tend to be of irregular tapered or pointed shapes. Such microholes behave as molds which produce tapered micropillars.

The following specific examples describing the manner and process of making and using the invention is intended to be illustrative rather than limiting. All parts are by weight unless indicated.

A polyacetal copolymer mandrel mold was shaped by turning on a computer numerical controlled (CNC) lathe. Subsequently, a pattern of blind microholes was formed into the surface of the mold by laser drilling. The holes in this example were approximately 250 μm in diameter, spaced about 500 μm from center to center and about 2500 μm deep. The shape of each hole is tapered, with the wide end at the surface. This laser drilled polyacetal copolymer mold was mounted by means of a threaded hole in the center of its posterior. A stainless steel fixture connected the mold to a holding plate which was placed on the elevator of a commercially available automatic dipping system.

The mold was lowered into a approximately 11% solids liquid silicone rubber dispersion, (Dow Corning Q7-2423 INT; a polydimethylsiloxane with platinum cure system dispersed in 1,1,1-trichloroethane) and raised from the liquid dispersion at controlled rates to coat the part. Sufficient coating thickness was formed by repeated dipping. The viscosity of the dispersion in this example was about 830 cp. The molds were dipped nine times to build sufficient thickness. The mandrel molds were rotated manually between dips to distribute the "drip" area. (The "drip" area is the area of a dip coated part in which the coating is the thickest, especially due to gravity flow. If the mandrels are not rotated or their dip-coated axis otherwise changed, the thick "drip" area for each dip will overlap providing a less uniform thickness across the entire envelope surface.) Approximately two hours elapsed between the ninth coat and the cue to provide ample time for the coating to dry by evaporation of the 1,1,1-trichlorethane solvent. Then the mandrel were placed in a preheated pressure oven (despatch LPB model). The coating was cured in the oven with a temperature of about 93°C. and gauge pressure of about 1.83 kg/cm² for a period of about 2 hours. After cooling, which was accelerated by blowing cool air across the mandrels, they were stripped. The envelopes removed from these mandrel forms contained micropillars, whose position corresponded to the mandrel holes. The height of these micropillars was about 750 μm.

While I have described certain specific embodiments of the invention for illustrative purposes, various modifications will be apparent to those skilled in the art which do not constitute departures from the spirit and scope of the invention as defined in the appended claims.

**Claims**

1. A method of manufacturing an envelope for a mammary prosthesis intended for implantation in a human breast which envelope is provided with a surface having outwardly projecting micropillars integral therewith comprising

(A) providing a three dimensional mandrel, the outer surface of which has an array of blind microholes therein having a depth of about 5 to 3500 microns and a diameter of about 5 to 3000 microns, said mandrel approximating the shape of the desired envelope and said microholes constituting a mold surface for formation of such micropillars,

(B) applying a crosslinkable elastomer-forming liquid dispersion of a polymer to said mandrel surface,

(C) subjecting said mandrel and polymer to an elevated pressure,

(D) curing said polymer while under elevated pressure,

(E) stripping the resultant elastomeric envelope from said mandrel, and

(F) turning said envelope inside out.

2. A process according to claim 1 wherein said elastomer is selected from the group consisting of polyurethane, polydimethylsiloxane and copolymers thereof.

3. A method of manufacturing an article for implantation in living tissue which article is provided with a surface having micropillars integral therewith and projecting outwardly from the surface thereof comprising

(A) providing a mold surface having an array of blind microholes thereon,

(B) applying a elastomer-forming liquid dispersion of a polymer to said mold surface,

(C) subjecting said mold and polymer to an elevated pressure,

(D) curing said polymer while under such elevated pressure, and

(E) stripping the resultant elastomeric article from said mold.

4. A process according to claim 3 wherein said polymer is further cured or crosslinked subsequent to step E.

5. A prosthesis made in accordance with claim 1.

6 An implantable device manufactured in accordance with the process of claim 3.

Fig. 1

Fig. 2

Fig. 3

Fig. 4

Fig. 5